# EUROPEAN PATENT SPECIFICATION

(11) **EP 1 416 911 B1**
(45) Date of publication and mention of the grant of the patent: **09.05.2007**
(21) Application number: 02747614.2
(22) Date of filing: 02.08.2002
(51) Int. Cl.: A61Q 17/00, A61Q 17/04, A61Q 19/00

(54) **PERSONAL CARE COMPOSITIONS**
KÖRPERPFLEGEZUSAMMENSETZUNGEN
COMPOSITIONS POUR SOINS PERSONNELS

(30) Priority: 11.08.2001 GB 0119639
(43) Date of publication of application: 12.05.2004
(73) Proprietor: The Boots Company PLC, Nottingham NG2 3AA (GB)
(72) Inventor: COOPER, Ryan, Nottingham NG2 3AA (GB); O'CONNOR, Clare, Nottingham NG2 3AA (GB); POPOW, Matthew, Nottingham NG2 3AA (GB); WHITBY, Daniel, Nottingham NG2 3AA (GB)
(74) Representative: Jones, Stephen Anthony
(86) International application number: PCT/GB2002/003576
(87) International publication number: WO 2003/013468

(56) References cited:
- EP-A- 0 685 223
- EP-A- 0 904 776
- EP-A- 1 034 778
- EP-A- 1 040 820
- EP-A- 1 050 296
- US-A- 6 096 294

## Description

The present invention relates to personal care compositions containing sunscreening compounds to counteract the deleterious effects of UV radiation. In one aspect of the present invention the personal care compositions are sunscreen compositions in which sunscreening compounds are used to protect the user's skin or hair from UV radiation. The term "sunscreen composition" is used herein to encompass sunscreening compositions such as moisturisers, day creams, colour cosmetics including foundations, lipsticks, eye shadows, blushers etc, tanning lotions and sunblockers, and toiletry products such as shower gels, shampoos and conditioners which are intended for topical application to provide protection for the skin or hair against the sun's rays or other sources of ultraviolet (UV) radiation. In a further aspect of the present invention the personal care compositions are cosmetic/toiletries compositions containing sunscreening compounds to protect the compositions from the deleterious effects of exposure of the compositions to UV radiation. The term "cosmetic/toiletries composition" is used herein to encompass compositions intended for application to the skin or hair in which the sunscreen compounds are present to protect the compositions from the deleterious effects of exposure of the composition to UV radiation. Examples of cosmetic/toiletries composition include gels such as bath gels or shower gels, shampoos optionally containing conditioning agents and/or anti-dandruff agents, hair conditioners, liquid soaps, creams and lotions. Such compositions may be emulsions (oil-in-water emulsions or water-in-oil emulsions).

In the formulation of a sunscreening composition, it is desirable to achieve a high sun protection factor (SPF). SPF is defined as the minimum dose of UV radiation required to show the first signs of burning or reddening of protected skin divided by the minimum dose for unprotected skin. The use of sunscreening compounds that provide the highest possible SPF gives the user of the composition the greatest protection. Alternatively, a given degree of protection can be achieved using lower concentrations of sunscreening compounds, with correspondingly reduced risks of irritation or sensitisation.

Another measure of the usefulness of a sunscreening product is the spectral width over which it is effective. This may be assessed in terms of industry standards such as the Boots Star Rating System, which is the ratio of the mean absorbance in the UVB region (around 280-320nm) to the mean absorbance in the UVA region (320-400nm). It is generally desirable that the product should absorb uniformly over both UVA and UVB regions. In known products, there may be less than the desired degree of absorption at wavelengths close to 400nm and there may also a trough in the absorbance profile at wavelengths around 330nm.

There have now been devised personal care compositions comprising combinations of several types of sunscreening compound in particular proportions that overcome or substantially mitigate the above-mentioned and/or other disadvantages of the prior art.

According to the invention, there are provided personal care compositions comprising
a) from 4 to 12 parts by weight of a dibenzoylmethane sunscreening compound;
b) from 3 to 9 parts by weight of a cyano-diphenyl acrylate sunscreening compound;
c) from 2.5 to 7.5 parts by weight of a salicylate sunscreening compound; and
d) from 0.5 to 1.5 parts by weight of a triazinic sunscreening compound.

The personal care compositions of the invention are advantageous primarily in that they exhibit relatively uniform absorption across the whole of the UVA and UVB spectral range. This provides important dermatological benefits in that it protects the skin across the whole range of UV wavelengths, and not just the UVB wavelengths that cause burning of the skin. Furthermore, the compositions may be synergistic in providing higher SPF's than those achieved with the individual sunscreening compounds alone. This enhances the protective effects of the compositions and/or permits a particular degree of protection to be achieved through the use of lower concentrations of sunscreening compounds. This in turn may lead to savings in cost, the active sunscreening compounds being relative expensive components of the compositions, and may also reduce the potential for adverse effects such as skin irritation and sensitisation.

The term "sunscreening compound" means a compound that absorbs radiation in a part of the wavelength range that is associated with the deleterious effects of sunlight, ie a compound that absorbs radiation in at least part of the wavelength range 280-400nm.

"Dibenzoylmethane" sunscreening compounds are sunscreening compounds that have a dibenzoylmethane grouping in their structure, irrespective of the nature of any substituents on that grouping. Dibenzoylmethane sunscreening compounds include 4-(1,1-dimethylethyl)-4'-methoxydibenzoyl methane (available commercially under the trade name PARSOL 1789 and Eusolex 9020) and 4-isopropyldibenzoylmethane (available commercially under the trade name EUSOLEX 8020), the former being particularly preferred.

"Cyano-diphenyl acrylate" sunscreening compounds are sunscreening compounds that are esters of cyanodiphenyl acrylic acid. Such compounds include alkyl α-cyano-β,β-diphenylacrylates such as octocrylene (2-ethylhexyl 2-cyano-3,3-diphenylacrylate) which is commercially available under the trade names Uvinul N539, Neo Heliopan 303, Escalol 597, Eusolex OCR and Parsol 340. Octocrylene is particularly preferred.

"Salicylate" sunscreening compounds are generally esters of salicylic acid or substituted derivatives thereof. Particularly preferred sunscreening compounds of this class are alkyl salicylate esters in which the alkyl group consists of a chain of 2 to 6 carbon atoms optionally substituted by a C₁₋₄ alkyl group, especially ethylhexyl salicylate which is commercially available under the trade names Eusolex OS, Neo Heliopan OS and Escalol 587.

"Triazinic" sunscreening compounds are sunscreening compounds with a structure incorporating a triazine moiety. Such compounds include triazines substituted at the 2-, 4- and 6- positions by groups -X-Ph-Y, in which X is -O- or -NH-, Ph is phenyl and Y is C₁₋₈alkyl, -O-C₁₋₈alkyl or -COO-C₁₋₈alkyl. Exemplary C₁₋₈alkyl groups include methyl, tert-butyl and 2-ethylhexyl. The Y group preferably occurs at the 4-position. Examples of such compounds include 2,4,6-trianilino-(p-carbo-2-ethylhexyl-1'-oxy)-1,3,5 triazine and bis-octyloxyphenol methoxyphenyl triazine. A particularly preferred triazinic sunscreening compound of this type is 4,6-bis[4-(2-ethylhexyloxycarbonyl)anilino]-2-[4-(tert-butylaminocarbonyl)anilino]-1,3,5-triazine, which is commonly known as diethylhexyl butamido triazone and is commercially available under the trade name UVASORB HEB.

We particularly prefer compositions in which
a) the dibenzoylmethane sunscreening compound is butyl methoxydibenzoylmethane;
b) the cyano-diphenyl acrylate sunscreening compound is octocrylene;
c) the salicylate sunscreening compound is ethylhexyl salicylate; and
d) the triazinic sunscreening compound is diethylhexyl butamido triazone.

Preferred proportions of the four classes of sunscreening compounds are:
a) from 6 to 10 parts by weight of dibenzoylmethane sunscreening compound;
b) from 4.5 to 7.5 parts by weight of cyano-diphenyl acrylate sunscreening compound;
c) from 3.5 to 6.5 parts by weight of salicylate sunscreening compound; and
d) from 0.75 to 1.25 parts by weight of triazinic sunscreening compound.

The stability of the compositions according to the invention may be enhanced by the inclusion of a photostabilising amount of a salicylate ester of formula I

(2-OH)Ph-(CO)-O-R I

wherein R represents an alkyl group consisting of a chain of from 7 to 16 carbon atoms substituted by at least one group selected from methyl and ethyl.

The group R may be represented by the generic structure la:

-Cₙ₋₁H₂ₙ₋₂₋ₘ(Sub)ₘ - CH₃

wherein n represents an integer from 7 to 16, m is 1 or more, and each group Sub independently represents a methyl or an ethyl group.

Preferably, m is an integer of between 1 and 4.

Where m is greater than 1, ie when there is more than one methyl and/or ethyl substituent on the alkyl chain, those methyl and/or ethyl groups may be attached to the same or different carbon atoms of the alkyl chain.

Where m is greater than 1, it is preferred that all the Sub groups in the molecule of formula I are the same, ie the Sub groups are either all methyl groups or all ethyl groups.

We particularly prefer compounds of formula I in which Sub represents methyl and m is from 1 to 4, particularly 3.

We particularly prefer compounds of formula I in which Sub represents ethyl and m is from 1 to 4, particularly 1.

Another preferred group of compounds are those in which n represents 7 to 12, particularly 7 to 10.

The group R preferably contains between 8 and 16 carbon atoms in total, more preferably between 9 and 14 carbon atoms, eg 10 or 13 carbon atoms.

Particular compounds of formula I that may be mentioned are isodecyl salicylate, in which R represents a 7-carbon chain substituted by 3 methyl groups, and isotridecyl salicylate, in which R represents a 10-carbon chain substituted by an ethyl group.

Compounds of formula I may all be used in the form of mixtures. Also, the compounds of formula I may be present in two or more isomeric forms. Thus, for instance, isodecyl salicylate may comprise molecules in which the three methyl groups are present at various positions on the alkyl chain. In such cases, a single molecular species may predominate, or two or more species may both be present in substantial proportions.

The personal care compositions of the present invention may contain 0.1 to 30% by weight of the salicylate ester of formula I, preferably 1 to 25%, more preferably 4 to 20%.

The personal care compositions of the present invention may contain a total 0.05 to 20% of sunscreening compounds by weight of the total composition. In compositions intended to be applied to the skin or hair to protect the skin or hair from the deleterious effects of exposure to UV radiation, the total amount of sunscreening compounds that may be present is preferably in the range 0.5 to 15%, more preferably 5 to 12%. In compositions in which the sunscreening compounds are present to protect the compositions from the deleterious effects of exposure to UV radiation, the total amount of sunscreening compounds that may be present is preferably less than 1% by weight of the total composition, more preferably 0.05 to 0.6%, most preferably 0.3 to 0.5%.

In compositions intended to be applied to the skin or hair to protect the skin or hair from the deleterious effects of exposure to UV radiation, the amounts of the various sunscreening compounds that are present are preferably as follows:
a) dibenzoylmethane sunscreening compound - preferably from 2 to 8%, more preferably 3 to 5%;
b) cyano-diphenyl acrylate sunscreening compound - preferably from 1.5 to 6%, more preferably 2 to 4%;
c) salicylate sunscreening compound - preferably from 1.25 to 5%, more preferably 1.5 to 3.5%;
d) triazinic sunscreening compound - preferably from 0.25 to 1%, more preferably 0.3 to 0.7%.

in compositions in which the sunscreening compounds are present to protect the compositions from the deleterious effects of exposure to UV radiation, the amounts of the various sunscreening compounds that are present are preferably as follows:
a) dibenzoylmethane sunscreening compound - preferably from 0.02 to 0.8%;
b) cyano-diphenyl acrylate sunscreening compound - preferably from 0.015 to 0.6%;
c) salicylate sunscreening compound - preferably from 0.0125 to 0.5%;
d) triazinic sunscreening compound - preferably from 0.0025 to 0.1%.

Example formulations include shampoos containing conditioning agents and pearlescent systems, hair conditioners, serums, creams, and lotions. Such compositions may be emulsions (oil-in-water or water-in-oil).

The sunscreening compounds of the present invention may be incorporated into sunscreen products such as aqueous or oily solutions or dispersions or emulsions in the conventional way. The emulsion may be an oil-in-water emulsion or a water-in-oil emulsion.

The oil phase of the water-in-oil or oil-in-water emulsions of the present invention may comprise for example:
a) hydrocarbon oils such as paraffin or mineral oils;
b) waxes such as beeswax or paraffin wax;
c) natural oils such as sunflower oil, apricot kernel oil, shea butter or jojoba oil;
d) silicone oils such as dimethicone, cyclomethicone or cetyldimethicone;
e) fatty acid esters such as isopropyl palmitate, isopropyl myristate or dioctylmaleate;
f) fatty alcohols such as cetyl alcohol or stearyl alcohol; or
g) mixtures thereof, for example, the blend of waxes available commercially under the trade name Cutina (Henkel).

In preferred water-in-oil compositions of the present invention the oil phase comprises 5 to 40%, more preferably 10 to 30% by weight of the composition. In preferred oil-in-water compositions of the present invention the oil phase comprises 5 to 30%, more preferably 10 to 20% by weight of the composition.

The emulsifiers used may be any emulsifiers known in the art for use in water-in-oil or oil-in-water emulsions. It has been found that particularly effective water-in-oil and oil-in-water sunscreen compositions can be prepared by using an emulsifier or mixture of emulsifiers selected from known cosmetically acceptable emulsifiers which include:
a) sesquioleates such as sorbitan sesquioleate, available commercially for example under the trade name Arlacel 83 (ICI), or polyglyceryl-2-sesquioleate;
b) ethoxylated esters of derivatives of natural oils such as the polyethoxylated ester of hydrogenated castor oil available commercially for example under the trade name Arlacel 989 (ICI);
c) silicone emulsifiers such as silicone polyols available commercially for example under the trade name ABIL WS08 (Th. Goldschmidt AG);
d) anionic emulsifiers such as fatty acid soaps e.g. potassium stearate and fatty acid sulphates e.g. sodium cetostearyl sulphate available commercially under the trade name Dehydag (Henkel);
e) ethoxylated fatty alcohols, for example the emulsifiers available commercially under the trade name Brij (ICI);
f) sorbitan esters, for example the emulsifiers available commercially under the trade name Span (ICI);
g) ethoxylated sorbitan esters, for example the emulsifiers available commercially under the trade name Tween (ICI);
h) ethoxylated fatty acid esters such as ethoxylated stearates, for example the emulsifiers available commercially under the trade name Myrj (ICI);
i) ethoxylated mono-, di-, and tri-glycerides, for example the emulsifiers available commercially under the trade name Labrafil (Alfa Chem.);
j) non-ionic self-emulsifying waxes, for example the wax available commercially under the trade name Polawax (Croda);
k) ethoxylated fatty acids, for example, the emulsifiers available commercially under the trade name Tefose (Alfa Chem.);
l) methylglucose esters such as polyglycerol-3 methyl glucose distearate available commercially under the name Tegocare 450 ( Degussa Goldschmidt); or
m) mixtures thereof.

The amount of emulsifier present in the emulsion compositions of the present invention is preferably in the range 1 to 10%.

The compositions of the present invention may additionally comprise other components which will be well known to those skilled in the art. These include, for example, emollients such as isopropyl myristate or triglycerides of fatty acids e.g. lauric triglyceride or capric/caprylic triglyceride, such as the triglyceride available commercially under the trade name Miglyol 810 (Huls UK); moisturisers such as D-panthenol; humectants such as glycerin or 1,3-butylene glycol; antioxidants such as DL-α-tocopherylacetate or butylated hydroxytoluene; emulsion stabilising salts such as sodium chloride, sodium citrate or magnesium sulphate; film formers to assist spreading on the surface of the skin such as alkylated polyvinylpyrrolidone, e.g. those available commercially under the trade name Antaron (GAF); thickeners such as acrylic acid polymers, e.g. those available commercially under the trade name Carbopol (B.F. Goodrich) or modified celluloses, e.g. hydroxyethylcellulose available commercially under the trade name Natrosol (Hercules) or alkylgalactomanans available under the trade name N-Hance; preservatives such as bronopol, sodium dehydroacetate, polyhexamethylenebiguanide hydrochloride, isothiazolone or diazolidinylurea; sequestering agents such as EDTA salts; perfumes and colourings.

The efficacy of the compositions of the invention may be assessed in terms of the Sun Protection Factor (SPF). The SPF may be measured *in vivo* by comparative measurements on volunteers under standardized conditions, eg the COLIPA industry-standard method. A method for the determination of the star rating of a composition, which also gives an *in vitro* indication of the likely SPF, is as follows:

### Measurement of Star Rating and in vitro SPF

The method is an *in vitro* assay conducted to measure the UVA/UVB absorbance ratio of a sunscreen product, to determine its star rating. It is based on a published method by Diffey and Robson ["A new substrate to measure sunscreen protection factors throughout the ultraviolet spectrum" BL Diffey and J Robson, J Soc Cosmet Chem, 40, 127-133 (May/June 1989)]. The UVA/UVB ratio is an indicator of the UVA absorbance properties of a sunscreen product, relative to UVB absorbance properties which enables classification for the star rating system. The higher the star rating the greater the ratio of UVA:UVB absorbance, a ratio of 0.8 or greater being classified as "4-star", 0.60 to 0.79 as "3-star", 0.40 to 0.59 as "2-star" and 0.20 to 0.39 as "1-star".

An SPF 290S analyser system was calibrated and optimised to ensure the maximum signal over the required 290 to 400 nm wavelength range, according to the operation manual. A blank UV transpore^{™} surgical tape is scanned as a reference to determine 100% transmission of UV light (290 nm - 400 nm). The sunscreen product is applied at a rate of 2 mg/cm² to transpore tape. The product is applied and is spread evenly over a total area of 114 cm². The product is then left to dry for 10 minutes prior to scanning. The tape with sunscreen on is then scanned in 12 different locations. A scan consists of UV transmission measurements taken at 5nm increments from 290 to 400 nm. At least 5 separate scans should be performed from which a mean star rating and in-vitro SPF can be calculated.

*In vitro* SPF's were measured for individual sunfilters in a standard formulation, to assess protection from burning, giving the following results:

| | |
|---|---|
| Butyl methoxydibenzoylmethane at 4% | SPF 6.14 |
| Octocrylene at 3% | SPF 5.09 |
| Ethylhexyl salicylate at 2.5% | SPF 2.97 |
| Diethylhexyl butamido triazone at 0.5% | SPF 3.19 |

Summing these results would give a total expected SPF in combination of 17.4 for a product containing all of the above filters at the same concentrations.

in fact, for a comparable formulation containing all of the above filters in combination, the measured SPF was 27.5. This represents an increase in efficiency of 63%

The invention will be illustrated by the following Examples which are given by way of example only. All percentages are by weight of the final composition.

Examples 1 to 3 are sun protection formulations intended to protect the user's skin from UV damage.

### Example 1

### Oil-in-Water Sun Protection Lotion

| Ingredient | | % |
|---|---|---|
| 1 | C12-C15 Alcohols Benzoate | 8 |
| 2 | Isotridecyl salicylate | 4 |
| 3 | Octocrylene | 3 |
| 4 | Butyl methoxydibenzoyl methane | 4.0 |
| 5 | Silicone Fluid 1000 | 2 |
| 6 | Microcrystalline Wax | 2.5 |
| 7 | DL-A-Tocopheryl Acetate | 0.2 |
| 8 | PVP/Hexadecene Copolymer | 1.75 |
| 9 | Glyceryl-3 Glucose Distearate | 2.5 |
| 10 | Acrylates/Vinyl Ester Copolymer | 0.15 |
| 11 | Ethylhexyl salicylate | 2.5 |
| 12 | Diethylhexyl butamido triazone | 0.5 |
| 13 | 1,3-Butylene Glycol | 5 |
| 14 | Sequestrene NA4 | 0.02 |
| 15 | Caustic Potash Solution 45% w/w | 0.068 |
| 16 | Preservative | qs |
| 17 | Water | to 100 |

### Method

Heat 1-9 and 11 to 70°C (Phase A).
Add 10 and homogenise for 5 minutes.
In a separate vessel, heat 13-17 to 70-75°C (Phase B).
Add Phase A to Phase B and homogenise for 10 minutes.
Cool to 30°C.
Make up to volume.

### Example 2

### Moisturising Day Cream Emulsion Containing Vitamins and Sunscreens

| | | |
|---|---|---|
| Ingredient | | % |
| 1 | Aqua | 69.5 |
| 2 | Butylene glycol | 5.0 |
| 3 | Isotridecyl salicylate | 4.0 |
| 4 | Paraffinum liquidum | 4.0 |
| 5 | Octocrylene | 3.0 |
| 6 | Ethylhexyl salicylate | 2.5 |
| 7 | Diethylhexyl butamido triazone | 0.5 |
| 8 | Petrolatum | 3.0 |
| 9 | Cetyl Alcohol | 2.0 |
| 10 | Glycerin | 2.0 |
| 11 | Dimethicone | 2.0 |
| 12 | Cetearyl alcohol | 1.6 |
| 13 | Sodium Ascorbyl Phosphate (Vitamin C) | 1.0 |
| 14 | Butyl methoxydibenzoylmethane | 4.0 |
| 15 | Hydroxyethylcellulose | 0.4 |
| 16 | PEG-20 stearate | 0.4 |
| 17 | Polyacrylamide | 0.40 |
| 18 | Parfum | 0.3 |
| 19 | C13-14 isoparaffin | 0.2 |
| 20 | Tocopheryl acetate (Vitamin E) | 0.15 |
| 21 | Retinyl palmitate (Vitamin A) | 0.12 |
| 22 | Tetrasodium EDTA | 0.1 |
| 23 | Citric acid | 0.08 |
| 24 | Laureth-7 | 0.055 |
| 25 | BHT | 0.0024 |
| 26 | Preservative | qs |

### Method

Heat 3-9, 11, 12, 14, 16, 19-21 and 25 to 70°C (Phase A).
In a separate vessel, heat 1, 2, 10, 22-24 and 26 to 70°C (Phase B).
To Phase B add 15 and homogenise for 20 minutes.
Adjust Phase A and Phase B to 70°C.
Add Phase A to Phase B and homogenise for 10 minutes.
Cool to 30°C.
Make up to volume.

### Example 3

### Water-in-Oil Emulsion

| Ingredient | | % |
|---|---|---|
| 1 | C12-C15 Alcohols Benzoate | 8 |
| 2 | Isotridecyl salicylate | 4 |
| 3 | Octocrylene | 3 |
| 4 | Butylmethoxy dibenzoyl methane | 4.0 |
| 5 | Silicone Fluid 1000 | 2 |
| 6 | Microcrystalline Wax | 2.5 |
| 7 | DL-A-Tocopheryl Acetate | 0.2 |
| 8 | PVP/Hexadecene Copolymer | 1.75 |
| 9 | Polyglyceryl-3-oleate | 1.75 |
| 10 | Cetyl Dimethicone Copolyol | 1.35 |
| 11 | Ethylhexyl salicylate | 2.5 |
| 12 | Diethylhexyl butamido triazone | 0.5 |
| 13 | 1,3-Butylene Glycol | 5 |
| 14 | Sequestrene NA4 | 0.02 |
| 15 | Sodium chloride | 0.75 |
| 16 | Magnesium sulphate | 0.75 |
| 17 | Preservative | qs |
| 18 | Water | to 100 |

### Method

Heat 1-12 to 70°C (Phase A).
In a separate vessel, heat 13-18 to 70°C (Phase B).
Stir Phase A into Phase B.
Homogenise for 10 minutes.
Cool to below 30°C.

Examples 4 to 6 are cosmetic formulations to protect the skin and hair from UV damage.

### Example 4

### Eye Cream

| Ingredient | % |
|---|---|
| Aqua | to 100 |
| Butylene glycol | 6 |
| Paraffinum liquidum | 5 |
| Octyl methoxycinnamate | 4 |
| Dimethicone | 2 |
| Petrolutum | 2 |
| Cetearyl octanoate | 1.8 |
| Cetearyl alcohol | 1.6 |
| Glyceryl stearate | 1.5 |
| Cetyl alcohol | 1 |
| Prunus dulcis | 1 |
| Glycerin | 0.57 |
| Hydrogenated vegetable glycerides citrate | 0.5 |
| Tocopheryl acetate | 0.5 |
| Bisabolol | 0.475 |
| Panthenol | 0.45 |
| Sodium phosphate | 0.42 |
| PEG-20 stearate | 0.4 |
| lsopropyl myristate | 0.2 |
| Carbomer | 0.15 |
| PEG-12 isostearate | 0.125 |
| Allantoin | 0.1 |
| Tetrasodium EDTA | 0.1 |
| Lactic acid | 0.088 |
| Disodium phophate | 0.083 |
| Potassium hydroxide | 0.051 |
| One or combination of the salicylates in Table 1 | 10.0 |
| Octocrylene | 3 |
| Butyl methoxydibenzoyl methane | 4.0 |
| Ethylhexyl salicylate | 2.5 |
| Diethylhexyl butamido triazone | 0.5 |
| Preservative | q.s |

**Table 1**

| |
|---|
| Isotridecyl salicylate |
| lsodecyl salicylate |

### Method

### Stage 1

Into the water, citric acid, EDTA, sodium phosphate, disodium phosphate and lactic acid are added and dispersed. Using a homogeniser, carbomer is added and hydrated. The aqueous phase is then heated to 70°C.

### Stage 2

The paraffinum liquidum, octyl methoxycinnamate, dimethicone, petrolatum, cetearyl octanoate, cetearyl alcohol, glyceryl stearate, cetyl alcohol, hydrogenated vegetable glycerides citrate, tocopheryl acetate, PEG-20 stearate, isopropyl myristate, salicylate, dibenzoyl methane and PEG-12 isostearate are mixed and heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the are then added and mixed. The product is then made to weight using purified water and is stirred until uniform.

### Example 5

### Foundation

| Ingredient | % |
|---|---|
| Aqua | to 100 |
| Butylene glycol | 9.8 |
| Cetearyl isononanoate | 4.9 |
| Dimethicone | 3.2 |
| Glycerin | 1.96 |
| Silica | 1.9 |
| Caprylic/capric triglyceride | 1.67 |
| Paraffinum liquidum | 1.67 |
| Petrolatum | 1.67 |
| Hydrogenated coco-glycerides | 1.67 |
| Cetearyl octanoate | 1.5 |
| Cetearyl alcohol | 1.35 |
| Octyl methoxycinnamate | 1.28 |
| Talc | 1 |
| Glyceryl stearate | 0.95 |
| PEG-100 stearate | 0.9 |
| Butyl methoxydibenzoylmethane | 0.6 |
| Saccharide isomerate | 0.54 |
| Lactic acid | 0.45 |
| Sodium polyacrylate | 0.45 |
| Boron nitride | 0.42 |
| Sodium PCA | 0.4 |
| Borago officinalis | 0.4 |
| Tocopheryl acetate | 0.4 |
| PVP/hexadecene copolymer | 0.4 |
| PEG-20 stearate | 0.33 |
| Glycolic acid | 0.2 |
| Sodium stearoyl lactylate | 0.2 |
| Isopropyl myristate | 0.17 |
| Polyaminopropyl biguanide | 0.16 |
| Tetrasodium EDTA | 0.1 |
| Xanthan gum | 0.1 |
| Citric acid | 0.06 |
| Alcohol denat. | 0.04 |
| Lecithin | 0.037 |
| Preservative | q.s |
| One or combination of the salicylates in Table 1 | 10.0 |
| Octocrylene | 3 |
| Butyl methoxydibenzoyl methane | 4.0 |
| Ethylhexyl salicylate | 2.5 |
| Diethylhexyl butamido triazone | 0.5 |

### Method

### Stage 1

Into the water, citric acid, EDTA and lactic acid are added and dispersed. Xanthan gum is pre-dispersed in butylene glycol and is added to the bulk. The aqueous phase is then heated to 70°C.

### Stage 2

The cetearyl isononanoate, dimethicone, silica, PVP/hexadecene copolymer, caprylic/capric triglyceride, paraffinum liquidum, petrolatum, hydrogenated coco-glycerides, cetearyl octanoate, cetearyl alcohol, octyl methoxycinnamate, talc, glyceryl stearate, PEG-100 stearate, butyl methoxydibenzoylmethane, borago officinalis, tocopheryl acetate, sodium stearoyl lactylate, isopropyl myristate, salicylate, dibenzoyl methane and lecithin oil phase are mixed and heated to 70°C to melt the waxes.

### Stage 3

Using a homogeniser, stage 2 is added to stage 1 and this is mixed until emulsified and uniform. The emulsion is then cooled to below 35°C using stirring. The remaining materials are then added and mixed. The product is then made to weight using purified water and is stirred until uniform.

### Example 6

### Lipstick

| Ingredient | % |
|---|---|
| Ricinus communis | 15 |
| Octyldodecanol | 10 |
| Pentaerythrityl tetracaprylate/caprate | 10 |
| Mica | 10 |
| Bis-diglyceryl caprylate/caprate/isostearate/ Stearate/hydroxystearate adipate | 7.5 |
| Paraffin | 5 |
| Cera microcristallina | 5 |
| Propylene glycol | 2 |
| Hydrogenated castor oil | 2 |
| Candelilla cera | 1 |
| Carnauba | 1 |
| Synthetic wax | 1 |
| Butyrospermum parkii | 1 |
| Titanium dioxide | 0.5 |
| Tocopheryl acetate | 0.2 |
| Polyquatemium-37 | 0.2 |
| Red colour | q.s |
| One or combination of the salicylates in Table 1 | 10.0 |
| Octocrylene | 3 |
| Butyl methoxydibenzoyl methane | 4.0 |
| Ethylhexyl salicylate | 2.5 |
| Diethylhexyl butamido triazone | 0.5 |

### Method

The materials are mixed in a vessel and heated to 85°C until melted and uniform. The product is cooled. The product poured into a suitable container and allowed to cool to room temperature to set.

Examples 7 to 10 are toiletry products designed to protect the hair and skin from UV damage to skin and hair lipids/proteins and natural and artificial hair colour.

### Example 7

### UV protective Bodv Wash

| Ingredient | % |
|---|---|
| Aqua | to 100 |
| Sodium lauryl sulfate | 20 |
| Cocamidopropyl betaine | 1.5 |
| Sodium chloride | 0.2 |
| PEG-6 Cocamide | 1 |
| Dipropylene glycol | 0.25 |
| PEG-18 glyceryl oleate/cocoate | 0.5 |
| PEG-40 hydrogenated castor oil | 0.5 |
| Polyquaternium-7 | 0.04 |
| PEG-7 glyceryl cocoate | 0.5 |
| Tetrasodium EDTA | 0.02 |
| Preservative | q.s |
| Parfum | q.s |
| One or a combination of the salicylates in Table 1 | 5 |
| Octocrylene | 1.5 |
| Butyl methoxydibenzoyl methane | 2.0 |
| Ethylhexyl salicylate | 1.25 |
| Diethylhexyl butamido triazone | 0.25 |

### Method

### Stage 1

The following materials were added to the water in order with stirring:
Tetrasodium EDTA, Sodium lauryl sulfate, sodium chloride, PEG-6, dipropylene glycol, PEG-18, PEG-40, PEG-7, cocamidopropyl betaine

### Stage 2

Maintaining stirring, the bulk was heated to 65°C. Once uniform, the bulk was cooled with constant stirring to below 35°C.

### Stage 3

The preservative, solvent, sunscreens and perfume were added and the product was made to weight with purified water. The product was stirred until cool and uniform.

### Example 8

### UV protective conditioning shampoo

| Ingredient | % |
|---|---|
| Aqua | to 100 |
| Alpha olefin sulfonate | 25 |
| Cocamide DEA | 2 |
| Lauramide DEA | 1 |
| Oleamide MIPA | 1.5 |
| Cocamidopropyl betaine | 2 |
| Oleth-3 phosphate | 0.1 |
| Lauric acid | 0.25 |
| Sodium chloride | 0.25 |
| Preservative | q.s |
| Parfum | q.s |
| One or a combination of the salicylates in Table 1 | 5 |
| Octocrylene | 1.5 |
| Butyl methoxydibenzoyl methane | 2.0 |
| Ethylhexyl salicylate | 1.25 |
| Diethylhexyl butamido triazone | 0.25 |

### Method

### Stage 1

The following materials were added to the water in order with stirring:
Alpha olefin sulfonate, cocamide DEA, lauramide DEA, oleamide MIPA, cocamidopropyl betaine, lauric acid, oleth-3 phosphate.

### Stage 2

Maintaining stirring, the bulk was heated to 65°C. Once uniform, the bulk was cooled with constant stirring to below 35°C.

### Stage 3

The preservative, solvent, sunscreens and perfume were added and the product was made to weight with purified water. The product was stirred until cool and uniform.

### Example 9

### UV protective hair conditioner

| Ingredient | % |
|---|---|
| Aqua | to 100 |
| Cetyl alcohol | 4 |
| Cocamide MEA | 2 |
| Stearamidopropyl dimethylamine | 1 |
| Centrimonium chloride | 0.5 |
| Citric acid | 0.02 |
| Lactic acid | 0.4 |
| Hydroxyethyl cellulose | 0.5 |
| Tetrasodium EDTA | 0.05 |
| Preservative | q.s. |
| Parfum | q.s |
| One or a combination of the salicylates in Table 1 | 5 |
| Octocrylene | 1.5 |
| Butyl methoxydibenzoyl methane | 2.0 |
| Ethylhexyl salicylate | 1.25 |
| Diethylhexyl butamido triazone | 0.25 |

### Method

### Stage 1

EDTA, citric acid and lactic acid were added to the water and dispersed with stirring. In sample 1, the hydroxyethyl cellulose was added and hydrated using a homogeniser for 5 minutes.

The other materials were added to the water in the order shown above (exluding the parfum and preservative) with stirring. With constant stirring, the bulk was heated to 70°C until all materials were dispersed, melted and uniform.

### Stage 2

The product was cooled to below 35°C using stirring. The preservative, solvent, sunscreens and perfume were.then added and the product was made to weight with purified water. The product was then stirred until cool and uniform.

### Example 10

### UV protective hair gel

| Ingredient | % |
|---|---|
| Aqua | to 100 |
| Carbomer 940 | 0.35 |
| Glycerin | 0.5 |
| Tetrasodium EDTA | 0.05 |
| Potassium Hydroxide | 0.06 |
| Preservative | q.s |
| One or a combination of the salicylates in Table 1 | 5 |
| Octocrylene | 1.5 |
| Butyl methoxydibenzoyl methane | 2.0 |
| Ethylhexyl salicylate | 1.25 |
| Diethylhexyl butamido triazone | 0.25 |

### Method

### Stage 1

EDTA was dispersed in the water using stirring. The Carbomer was then added and hydrated using homogenisation for 30 minutes..

Glycerin was then added and mixed until uniform using stirring.

### Stage 2

The preservative, solvent and sunscreens were then added and made to weight with purified water. The gel was stirred until uniform.

Examples 11 to 14 are formulations in which the combinations of sunscreens are used to protect the formula itself from UV degradation.

### Example 11

### Body Wash

| Ingredient | % |
|---|---|
| Aqua | to 100 |
| Sodium lauryl sulfate | 20 |
| Cocamidopropyl betaine | 1.5 |
| Sodium chloride | 0.2 |
| PEG-6 Cocamide | 1 |
| Dipropylene glycol | 0.25 |
| PEG-18 glyceryl oleate/cocoate | 0.5 |
| PEG-40 hydrogenated castor oil | 0.5 |
| Polyquaternium-7 | 0.04 |
| PEG-7 glyceryl cocoate | 0.5 |
| Tetrasodium EDTA | 0.02 |
| Preservative | q.s |
| Parfum | q.s |
| One or a combination of the salicylates in Table 1 | 0.5 |
| Octocrylene | 0.15 |
| Butyl methoxydibenzoyl methane | 0.20 |
| Ethylhexyl salicylate | 0.125 |
| Diethylhexyl butamido triazone | 0.025 |

### Method

### Stage 1

The following materials were added to the water in order with stirring:
(in sample 1, polyquaternium-7 was added first) Tetrasodium EDTA, Sodium lauryl sulfate, sodium chloride, PEG-6, dipropylene glycol, PEG-18, PEG-40, PEG-7, cocamidopropyl betaine

### Stage 2

Maintaining stirring, the bulk was heated to 65°C. Once uniform, the bulk was cooled with constant stirring to below 35°C.

### Stage 3

The preservative, solvent, sunscreens and perfume were added and the product was made to weight with purified water. The product was stirred until cool and uniform.

### Example 12

### Conditioning Shampoo

| Ingredient | % |
|---|---|
| Aqua | to 100 |
| Alpha olefin sulfonate | 25 |
| Cocamide DEA | 2 |
| Lauramide DEA | 1 |
| Oleamide MIPA | 1.5 |
| Cocamidopropyl betaine | 2 |
| Oleth-3 phosphate | 0.1 |
| Lauric acid | 0.25 |
| Sodium chloride | 0.25 |
| Preservative | q.s |
| Parfum | q.s |
| One or a combination of the salicylates | |
| in Table 1 | 0.5 |
| Octocrylene | 0.15 |
| Butyl methoxydibenzoyl methane | 0.20 |
| Ethylhexyl salicylate | 0.125 |
| Diethylhexyl butamido triazone | 0.025 |

### Method

### Stage 1

The following materials were added to the water in order with stirring:
Alpha olefin sulfonate, cocamide DEA, lauramide DEA, oleamide MIPA, cocamidopropyl betaine, lauric acid, oleth-3 phosphate.

### Stage 2

Maintaining stirring, the bulk was heated to 65°C. Once uniform, the bulk was cooled with constant stirring to below 35°C.

### Stage 3

The preservative, solvent, sunscreens and perfume were added and the product was made to weight with purified water. The product was stirred until cool and uniform.

### Example 13

### Hair conditioner

| Ingredient | % |
|---|---|
| Aqua | to 100 |
| Cetyl alcohol | 4 |
| Cocamide MEA | 2 |
| Stearamidopropyl dimethylamine | 1 |
| Centrimonium chloride | 0.5 |
| Citric acid | 0.02 |
| Lactic acid | 0.4 |
| Hydroxyethyl cellulose | 0.5 |
| Tetrasodium EDTA | 0.05 |
| Preservative | q.s |
| Parfum | q.s |
| One or a combination of the salicylates in Table 1 | 0.5 |
| Octocrylene | 0.15 |
| Butyl methoxydibenzoyl methane | 0.20 |
| Ethylhexyl salicylate | 0.125 |
| Diethylhexyl butamido triazone | 0.025 |

### Method

### Stage 1

EDTA, citric acid and lactic acid were added to the water and dispersed with stirring. In sample 1, the hydroxyethyl cellulose was added and hydrated using a homogeniser for 5 minutes.

The other materials were added to the water in the order shown above (exluding the parfum and preservative) with stirring. With constant stirring, the bulk was heated to 70°C until all materials were dispersed, melted and uniform.

### Stage 2

The product was cooled to below 35°C using stirring. The preservative, solvent , sunscreens and perfume were then added and the product was made to weight with purified water. The product was then stirred until cool and uniform.

### Example 14

### Hair gel

| Ingredient | % |
|---|---|
| Aqua | to 100 |
| Carbomer 940 | 0.35 |
| Glycerin | 0.5 |
| Tetrasodium EDTA | 0.05 |
| Potassium Hydroxide | 0.06 |
| Preservative | q.s |
| One or a combination of the salicylates in Table 1 | 0.5 |
| Octocrylene | 0.15 |
| Butyl methoxydibenzoyl methane | 0.20 |
| Ethylhexyl salicylate | 0.125 |
| Diethylhexyl butamido triazone | 0.025 |

### Method

### Stage 1

EDTA was dispersed in the water using stirring. The Carbomer was then added and hydrated using homogenisation for 30 minutes..

Glycerin was then added and mixed until uniform using stirring.

### Stage 2

The preservative, solvent and sunscreens were then added and they were made to weight with purified water. The gel was stirred until uniform.

## Claims

1. A personal care composition comprising
a) from 4 to 12 parts by weight of a dibenzoylmethane sunscreening compound;
b) from 3 to 9 parts by weight of a cyano-diphenyl acrylate sunscreening compound;
c) from 2.5 to 7.5 parts by weight of a salicylate sunscreening compound; and
d) from 0.5 to 1.5 parts by weight of a triazinic sunscreening compound.

2. A composition as claimed in Claim 1, wherein the dibenzoylmethane sunscreening compound is selected from the group consisting of 4-(1,1-dimethylethyl)-4'-methoxydibenzoyl methane and 4-isopropyldibenzoylmethane.

3. A composition as claimed in Claim 2, wherein the dibenzoylmethane sunscreening compound is 4-(1,1-dimethylethyl)-4'-methoxydibenzoyl methane.

4. A composition as claimed in any preceding claim, wherein the cyano-diphenyl acrylate sunscreening compound is an alkyl α-cyano-β,β-diphenylacrylate.

5. A composition as claimed in Claim 4, wherein the cyano-diphenyl acrylate sunscreening compound is 2-ethylhexyl 2-cyano-3,3-diphenylacrylate.

6. A composition as claimed in any preceding claim, wherein the salicylate sunscreening compound is an alkyl salicylate ester in which the alkyl group consists of a chain of 2 to 6 carbon atoms, optionally substituted by a C₁₋₄ alkyl group.

7. A composition as claimed in Claim 6, wherein the salicylate sunscreening compound is ethylhexyl salicylate

8. A composition as claimed in any preceding claim, wherein the triazinic sunscreening compound is a triazine substituted at the 2-, 4- and 6- positions by groups -X-Ph-Y, in which X- is -O- or -NH-, Ph is phenyl and Y is -C₁₋₈alkyl, -O-C₁₋₈alkyl or -COOC₁₋₈alkyl.

9. A composition as claimed in Claim 8, wherein the triazine sunscreening compound is 4,6-bis[4-(2-ethylhexyloxycarbonyl)anilino]-2-[4-(tert-butylaminocarbonyl)anilino]-1,3,5-triazine.

10. A composition as claimed in Claim 1, wherein
a) the dibenzoylmethane sunscreening compound is butyl methoxydibenzoylmethane;
b) the cyano-diphenyl acrylate sunscreening compound is octocrylene;
c) the salicylate sunscreening compound is ethylhexyl salicylate; and
d) the triazinic sunscreening compound is diethylhexyl butamido triazone.

11. A composition as claimed in any preceding claim, which comprises from 6 to 10 parts by weight of dibenzoylmethane sunscreening compound.

12. A composition as claimed in any preceding claim, which comprises from 4.5 to 7.5 parts by weight of cyano-diphenyl acrylate sunscreening compound.

13. A composition as claimed in any preceding claim, which comprises from 3.5 to 6.5 parts by weight of salicylate sunscreening compound.

14. A composition as claimed in any preceding claim, which comprises from 0.75 to 1.25 parts by weight of triazinic sunscreening compound.

15. A composition as claimed in any preceding claim, which further comprises a photostabilising amount of a salicylate ester of formula I
(2-OH)Ph-(CO)-O-R I
wherein R represents
-Cₙ₋₁H₂ₙ₋₂₋ₘ(Sub)ₘ - CH₃
in which n represents an integer from 7 to 16, m is 1 or more, and each group Sub independently represents a methyl or an ethyl group.

16. A composition as claimed in Claim 15, wherein Sub represents methyl and m is from 1 to 4, particularly 3.

17. A composition as claimed in Claim 15, wherein Sub represents ethyl and m is from 1 to 4, particularly 1.

18. A composition as claimed in Claim 15, wherein the compound of formula I is isodecyl salicylate or isotridecyl salicylate.

19. A composition as claimed in any one of Claims 15 to 18, which comprises from 0.1 to 30% by weight of the salicylate ester of formula I.

20. A composition as claimed in any preceding claim, which contains a total 0.05 to 20% of sunscreening compounds by weight of the total composition.

21. A composition as claimed in Claim 1, which is intended to be applied to the skin or hair to protect the skin or hair from the deleterious effects of exposure to UV radiation, and in which the total amount of sunscreening compounds present is in the range 0.5 to 15% by weight.

22. A composition as claimed in Claim 21, in which the amounts of the various sunscreening compounds that are present are:
a) dibenzoylmethane sunscreening compound - from 2 to 8%, more preferably 3 to 5%;
b) cyano-diphenyl acrylate sunscreening compound - from 1.5 to 6%, more preferably 2 to 4%;
c) salicylate sunscreening compound - from 1.25 to 5%, more preferably 1.5 to 3.5%;
d) triazinic sunscreening compound - from 0.25 to 1%, more preferably 0.3 to 0.7%.

23. A composition as claimed in Claim 1, in which the sunscreening compounds are present to protect the compositions from the deleterious effects of exposure to UV radiation, and the total amount of sunscreening compounds present is less than 1% by weight of the total composition.

24. A composition as claimed in Claim 23, wherein the the amounts of the various sunscreening compounds are:
a) dibenzoylmethane sunscreening compound -from 0.02 to 0.8%;
b) cyano-diphenyl acrylate sunscreening compound -from 0.015 to 0.6%;
c) salicylate sunscreening compound -from 0.0125 to 0.5%;
d) triazinic sunscreening compound -from 0.0025 to 0.1%.

25. A composition as claimed in any preceding claim, which is an emulsion.

26. A composition as claimed in Claim 25, which is an oil-in-water emulsion.

27. A composition as claimed in Claim 25, which is a water-in-oil emulsion.

28. A composition as claimed in any one of claims 25 to 27, wherein the oil phase comprises one or more of :
a) hydrocarbon oils;
b) waxes;
c) natural oils;
d) silicone oils;
e) fatty acid esters; and
f) fatty alcohols.

29. A composition as claimed in Claim 27, wherein the oil phase comprises 5 to 40% by weight of the composition.

30. A composition as claimed in Claim 26, wherein the oil phase comprises 5 to 30% by weight of the composition.

31. A composition as claimed in any one of Claims 25 to 30, which further comprises one or more emulsifiers selected from the group consisting of:
a) sesquioleates;
b) ethoxylated esters of derivatives of natural oils;
c) silicone emulsifiers;
d) anionic emulsifiers;
e) ethoxylated fatty alcohols;
f) sorbitan esters;
g) ethoxylated sorbitan esters;
h) ethoxylated fatty acid esters;
i) ethoxylated mono-, di-, and tri-glycerides;
j) non-ionic self-emulsifying waxes;
k) ethoxylated fatty acids; and
l) methylglucose esters.

32. A composition as claimed in Claim 31, wherein the amount of emulsifier present in the composition is in the range 1 to 10% by weight.

## Patentansprüche

1. Körperpflegezusammensetzung, umfassend
a) 4 bis 12 Gewichtsteile von einer Dibenzoylmethan-Sonnenschutzverbindung;
b) 3 bis 9 Gewichtsteile von einer Cyano-diphenylacrylat-Sonnenschutzverbindung;
c) 2,5 bis 7,5 Gewichtsteile von einer Salicylat-Sonnenschutzverbindung; und
d) 0,5 bis 1,5 Gewichtsteile von einer Triazin-Sonnenschutzverbindung.

2. Zusammensetzung nach Anspruch 1, worin die Dibenzoylmethan-Sonnenschutzverbindung ausgewählt ist aus der Gruppe, bestehend aus 4-(1,1-Dimethylethyl)-4'-methoxydibenzoylmethan und 4-Isopropyldibenzoylmethan.

3. Zusammensetzung nach Anspruch 2, worin die Dibenzoylmethan-Sonnenschutzverbindung 4-(1,1-Dimethylethyl)-4'-methoxydibenzoylmethan darstellt.

4. Zusammensetzung nach einem vorangehenden Anspruch, worin die Cyanodiphenylacrylat-Sonnenschutzverbindung ein α-Cyano-β,β-diphenylacrylsäurealkylester ist.

5. Zusammensetzung nach Anspruch 4, worin die Cyano-diphenylacrylat-Sonnenschutzverbindung 2-Cyano-3,3-diphenylacrylsäure-2-ethylhexylester ist.

6. Zusammensetzung nach einem vorangehenden Anspruch, worin die Salicylat-Sonnenschutzverbindung ein Salicylsäurealkylester ist, worin die Alkylgruppe aus einer Kette von 2 bis 6 Kohlenstoffatomen, gegebenenfalls substituiert mit einer C₁₋₄-Alkylgruppe, besteht.

7. Zusammensetzung nach Anspruch 6, worin die Salicylat-Sonnenschutzverbindung Salicylsäureethylhexylester ist.

8. Zusammensetzung nach einem vorangehenden Anspruch, worin die Triazin-Sonnenschutzverbindung ein Triazin, substituiert an den 2-, 4- und 6-Positionen durch Gruppen -X-Ph-Y, darstellt, worin X- -O- oder -NH- darstellt, Ph Phenyl darstellt und Y -C₁₋₈-Alkyl, -O-C₁₋₈-Alkyl oder -COOC₁₋₈-Alkyl darstellt.

9. Zusammensetzung nach Anspruch 8, worin die Triazin-Sonnenschutzverbindung 4,6-Bis[9-(2-ethylhexyloxycarbonyl)anilino]-2-[4-(tert-butylaminocarbonyl)anilino]-1,3,5-triazin ist.

10. Zusammensetzung nach Anspruch 1, worin
a) die Dibenzoylmethan-Sonnenschutzverbindung Butylmethoxydibenzoylmethan darstellt;
b) die Cyano-diphenylacrylat-Sonnenschutzverbindung Octocrylen darstellt;
c) die Salicylat-Sonnenschutzverbindung Salicylsäureethylhexylester darstellt; und
d) die Triazin-Sonnenschutzverbindung Diethylhexylbutamidotriazon darstellt.

11. Zusammensetzung nach einem vorangehenden Anspruch, die 6 bis 10 Gewichtsteile Dibenzoylmethan-Sonnenschutzverbindung umfasst.

12. Zusammensetzung nach einem vorangehenden Anspruch, die 4,5 bis 7,5 Gewichtsteile Cyano-diphenylacrylat-Sonnenschutzverbindung umfasst.

13. Zusammensetzung nach einem vorangehenden Anspruch, die 3,5 bis 6,5 Gewichtsteile Salicylat-Sonnenschutzverbindung umfasst.

14. Zusammensetzung nach einem vorangehenden Anspruch, die 0,75 bis 1,25 Gewichtsteile Triazin-Sonnenschutzverbindung umfasst.

15. Zusammensetzung nach einem vorangehenden Anspruch, die weiterhin eine photostabilisierende Menge eines Salicylatesters der Formel I
(2-OH)Ph-(CO)-O-R I,
worin R
-Cₙ₋₁H₂ₙ₋₂₋ₘ(Sub)ₘ - CH₃
wiedergibt,
worin n eine ganze Zahl von 7 bis 16 wiedergibt, m 1 oder mehr ist und jede Gruppe Sub unabhängig eine Methyl- oder eine Ethylgruppe wiedergibt, umfasst.

16. Zusammensetzung nach Anspruch 15, worin Sub Methyl wiedergibt und m 1 bis 4, insbesondere 3, ist.

17. Zusammensetzung nach Anspruch 15, worin Sub Ethyl wiedergibt und m 1 bis 4, insbesondere 1, ist.

18. Zusammensetzung nach Anspruch 15, worin die Verbindung der Formel I Salicylsäureisodecylester oder Salicylsäureisotridecylester ist.

19. Zusammensetzung nach einem der Ansprüche 15 bis 18, die 0,1 bis 30 Gewichtsprozent des Salicylatesters der Formel I umfasst.

20. Zusammensetzung nach einem vorangehenden Anspruch, die insgesamt 0,05 bis 20 % Sonnenschutzverbindungen, auf das Gewicht der Gesamtzusammensetzung, enthält.

21. Zusammensetzung nach Anspruch 1, die vorgesehen ist, auf die Haut oder das Haar aufgetragen zu werden, um die Haut oder das Haar vor den verschlechternden Wirkungen der Aussetzung von UV-Strahlung zu schützen, und worin die Gesamtmenge an vorliegenden Sonnenschutzverbindungen im Bereich 0,5 bis 15 Gewichtsprozent liegt.

22. Zusammensetzung nach Anspruch 21, worin die Mengen der verschiedenen vorliegenden Sonnenschutzverbindungen sind:
a) Dibenzoylmethan-Sonnenschutzverbindung - 2 bis 8 %, bevorzugter 3 bis 5 %;
b) Cyano-diphenylacrylat-Sonnenschutzverbindung - 1,5 bis 6 %, bevorzugter 2 bis 4 %;
c) Salicylat-Sonnenschutzverbindung - 1,25 bis 5 %, bevorzugter 1,5 bis 3,5 %;
d) Triazin-Sonnenschutzverbindung - 0,25 bis 1 %, bevorzugter 0,3 bis 0,7 %.

23. Zusammensetzung nach Anspruch 1, worin die Sonnenschutzverbindungen vorliegen, um die Zusammensetzungen vor den verschlechternden Wirkungen der Aussetzung von UV-Strahlung zu schützen, und die Gesamtmenge an vorliegenden Sonnenschutzverbindungen weniger als 1 Gewichtsprozent der Gesamtzusammensetzung ist.

24. Zusammensetzung nach Anspruch 23, worin die Mengen der verschiedenen Sonnenschutzverbindungen sind:
a) Dibenzoylmethan-Sonnenschutzverbindung - 0,02 bis 0,8 %;
b) Cyano-diphenylacrylat-Sonnenschutzverbindung - 0,015 bis 0,6 %,
c) Salicylat-Sonnenschutzverbindung - 0,0125 bis 0,5 %;
d) Triazin-Sonnenschutzverbindung - 0,0025 bis 0,1 %.

25. Zusammensetzung nach einem vorangehenden Anspruch, die eine Emulsion darstellt.

26. Zusammensetzung nach Anspruch 25, die eine Öl-in-Wasser-Emulsion darstellt.

27. Zusammensetzung nach Anspruch 25, die eine Wasser-in-Öl-Emulsion darstellt.

28. Zusammensetzung nach einem der Ansprüche 25 bis 27, worin die Ölphase einen oder mehrere umfasst von:
a) Kohlenwasserstoffölen;
b) Wachsen;
c) natürlichen Ölen;
d) Silikonölen;
e) Fettsäureestern und
f) Fettalkoholen.

29. Zusammensetzung nach Anspruch 27, worin die Ölphase 5 bis 40 Gewichtsprozent der Zusammensetzung umfasst.

30. Zusammensetzung nach Anspruch 26, worin die Ölphase 5 bis 30 Gewichtsprozent der Zusammensetzung umfasst.

31. Zusammensetzung nach einem der Ansprüche 25 bis 30, die weiterhin einen oder mehrere Emulgatoren umfasst, ausgewählt aus der Gruppe, bestehend aus:
a) Sesquioleaten;
b) ethoxylierten Estern von Derivaten von natürlichen Ölen;
c) Silikonemulgatoren;
d) anionischen Emulgatoren;
e) ethoxylierten Fettalkoholen;
f) Sorbitanestern;
g) ethoxylierten Sorbitanestern;
h) ethoxylierten Fettsäureestern;
i) ethoxylierten Mono-, Di- und Triglyceriden;
j) nichtionischen selbstemulgierenden Wachsen;
k) ethoxylierten Fettsäuren und
l) Methylglucoseestern.

32. Zusammensetzung nach Anspruch 31, worin die Menge an in der Zusammensetzung vorliegendem Emulgator im Bereich von 1 bis 10 Gewichtsprozent liegt.

## Revendications

1. Composition pour soins personnels comprenant:
a) de 4 à 12 parties en poids d'un composé d'écran solaire de type dibenzoylméthane ;
b) de 3 à 9 parties en poids d'un composé d'écran solaire de type cyano-diphénylacrylate ;
c) de 2,5 à 7,5 parties en poids d'un composé d'écran solaire de type salicylate; et
d) de 0,5 à 1,5 parties en poids d'un composé d'écran solaire triazinique.

2. Composition selon la revendication 1, dans laquelle le composé d'écran solaire de type dibenzoylméthane est choisi dans le groupe comprenant le 4-(1,1-diméthyléthyle)-4'-méthoxydibenzoylméthane et le 4-isopropyldibenzoylméthane.

3. Composition selon la revendication 2, dans laquelle le composé d'écran solaire de type dibenzoylméthane est le 4-(1,1-diméthyléthyle)-4'-méthoxydibenzoylméthane.

4. Composition selon l'une quelconque des revendications précédentes, dans laquelle le composé d'écran solaire de type cyano-diphénylacrylate est un α-cyano-β,β-diphénylacrylate d'alkyle.

5. Composition selon la revendication 4, dans laquelle le composé d'écran solaire de type cyano-diphénylacrylate est le 2-cyano-3,3-diphénylacrylate de 2-éthylhexyle.

6. Composition selon l'une quelconque des revendications précédentes, dans laquelle le composé d'écran solaire de type salicylate est un ester de type salicylate d'alkyle dans lequel le groupe alkyle consiste en une chaîne de 2 à 6 atomes de carbone, facultativement substitués par un groupe alkyle en C₁ à C₄.

7. Composition selon la revendication 6, dans laquelle le composé d'écran solaire de type salicylate est le salicylate d'éthylhexyle.

8. Composition selon l'une quelconque des revendications précédentes, dans laquelle le composé d'écran solaire triazinique est une triazine substituée au niveau des positions 2, 4 et 6 par des groupes -X-Ph-Y, dans lesquels X- est -O- ou -NH-, Ph est un phényle et Y est un alkyle en C₁ à C₈, -O-(alkyle en C₁ à C₈) ou -COO(alkyle en C₁ à C₈).

9. Composition selon la revendication 8, dans laquelle le composé d'écran solaire de type triazine est la 4,6-bis[4-(2-éthylhexyloxycarbonyle) anilino]-2-[4-(tert-butylaminocarbonyle) anilino]-1,3,5-triazine.

10. Composition selon la revendication 1, dans laquelle
a) le composé d'écran solaire de type dibenzoylméthane est le butylméthoxydibenzoylméthane ;
b) le composé d'écran solaire de type cyano-diphénylacrylate est l'octocrylène ;
c) le composé d'écran solaire de type salicylate est le salicylate d'éthylhexyle ; et
d) le composé d'écran solaire triazinique est la diéthylhexyle butamido triazone.

11. Composition selon l'une quelconque des revendications précédentes, qui comprend de 6 à 10 parties en poids de composé d'écran solaire de type dibenzoylméthane.

12. Composition selon l'une quelconque des revendications précédentes, qui comprend de 4,5 à 7,5 parties en poids de composé d'écran solaire de type cyano-diphénylacrylate.

13. Composition selon l'une quelconque des revendications précédentes, qui comprend de 3,5 à 6,5 parties en poids de composé d'écran solaire de type salicylate.

14. Composition selon l'une quelconque des revendications précédentes, qui comprend de 0,75 à 1,25 parties en poids de composé d'écran solaire triazinique.

15. Composition selon l'une quelconque des revendications précédentes, qui comprend en outre une quantité photostabilisante d'un ester de type salicylate de formule I:
(2-OH)Ph-(CO)-O-R I
dans laquelle R représente
-Cₙ₋₁H₂ₙ₋₂₋ₘ(Sub)ₘ-CH₃
où n représente un nombre entier de 7 à 16, m est égal à 1 ou plus, et chaque groupe Sub représente indépendamment un groupe méthyle ou éthyle.

16. Composition selon la revendication 15, dans laquelle Sub représente un méthyle et m va de 1 à 4, en particulier égal à 3.

17. Composition selon la revendication 15, dans laquelle Sub représente un éthyle et m va de 1 à 4, en particulier égal à 1.

18. Composition selon la revendication 15, dans laquelle le composé de formule I est le salicylate d'isodécyle ou le salicylate d'isotridécyle.

19. Composition selon l'une quelconque des revendications 15 à 18, qui comprend de 0,1 à 30 % en poids de l'ester de type salicylate de formule I.

20. Composition selon l'une quelconque des revendications précédentes, qui contient un total de 0,05 à 20 % de composés d'écran solaire en poids de la composition totale.

21. Composition selon la revendication 1, qui est destinée à être appliquée à la peau ou aux cheveux pour protéger la peau ou les cheveux des effets nuisibles de l'exposition à la radiation UV, et dans laquelle la quantité totale des composés d'écran solaire présents est dans la gamme de 0,5 à 15 % en poids.

22. Composition selon la revendication 21, dans laquelle les quantités des différents composés d'écran solaire qui sont présents sont:
a) composé d'écran solaire de type dibenzoylméthane : de 2 à 8 %, de préférence de 3 à 5 % ;
b) composé d'écran solaire de type cyano-diphénylacrylate : de 1,5 à 6 %, de préférence de 2 à 4 % ;
c) composé d'écran solaire de type salicylate : de 1,25 à 5 %, de préférence de 1,5 à 3,5 % ;
d) composé d'écran solaire triazinique: de 0,25 à 1 %, de préférence de 0,3 à 0,7 %.

23. Composition selon la revendication 1, dans laquelle les composés d'écran solaire sont présents pour protéger les compositions des effets nuisibles de l'exposition à la radiation UV, et la quantité totale des composés d'écran solaire présents est inférieure à 1 % en poids de la composition totale.

24. Composition selon la revendication 23, dans laquelle les quantités des différents composés d'écran solaire sont :
a) composé d'écran solaire de type dibenzoylméthane: de 0,02 à 0,8 % ;
b) composé d'écran solaire de type cyano-diphénylacrylate: de 0,015 à 0,6%;
c) composé d'écran solaire de type salicylate : de 0,0125 à 0,5 % ;
d) composé d'écran solaire triazinique : de 0,0025 à 0,1 %.

25. Composition selon l'une quelconque des revendications précédentes, qui est une émulsion.

26. Composition selon la revendication 25, qui est une émulsion d'huile dans l'eau.

27. Composition selon la revendication 25, qui est une émulsion d'eau dans l'huile.

28. Composition selon l'une quelconque des revendications 25 à 27, dans laquelle la phase huileuse comprend un ou plusieurs éléments parmi:
a) les huiles hydrocarbonées ;
b) les cires ;
c) les huiles naturelles;
d) les huiles siliconées ;
e) les esters d'acides gras ; et
f) les alcools gras.

29. Composition selon la revendication 27, dans laquelle la phase huileuse comprend de 5 à 40 % en poids de la composition.

30. Composition selon la revendication 26, dans laquelle la phase huileuse comprend de 5 à 30 % en poids de la composition.

31. Composition selon l'une quelconque des revendications 25 à 30, qui comprend en outre un ou plusieurs émulsifiants choisis dans le groupe comprenant :
a) les sesquioléates ;
b) les esters éthoxylés de dérivés d'huiles naturelles ;
c) les émulsifiants siliconés ;
d) les émulsifiants anioniques ;
e) les alcools gras éthoxylés ;
f) les esters de sorbitan ;
g) les esters de sorbitan éthoxylés ;
h) les esters d'acides gras éthoxylés ;
i) les mono-, di-, et tri-glycérides éthoxylés ;
j) les cires auto-émulsifiantes non ioniques;
k) les acides gras éthoxylés ; et
l) les esters de méthylglucose.

32. Composition selon la revendication 31, dans laquelle la quantité d'émulsifiant présent dans la composition est dans la gamme de 1 à 10 % en poids.
